# EUROPEAN PATENT SPECIFICATION

(11) **EP 4 301 313 B1**
(45) Date of publication and mention of the grant of the patent: **27.08.2025**
(21) Application number: 22787196.9
(22) Date of filing: 12.04.2022
(51) Int. Cl.: B05B 11/10, B05B 11/02, B05B 15/25, A45D 34/00, B01F 27/1151, B01F 27/805, B01F 27/93, B01F 29/10, B65D 47/34, B05B 11/00, A61M 35/00, A45D 40/00

(54) **METHODS AND SYSTEMS FOR COMPOSITION COMPOUNDING**
VERFAHREN UND SYSTEME ZUR ZUSAMMENSETZUNGSZUSAMMENSTELLUNG
PROCÉDÉS ET SYSTÈMES DE MÉLANGE DE COMPOSITION

(30) Priority: 13.04.2021 US 202163174231 P
(43) Date of publication of application: 10.01.2024
(73) Proprietor: MEDISCA PHARMACEUTIQUE INC., St-Laurent, QC H4R 3A6 (CA)
(72) Inventor: DANOPOULOS, Panagiota, Québec H4R 3A6 (CA)
(74) Representative: Bird & Bird LLP - Hamburg
(86) International application number: PCT/CA2022/050566
(87) International publication number: WO 2022/217352

(56) References cited:
- WO-A1-2018/085942
- WO-A1-2019/032756
- KR-A- 20110 016 832
- KR-B1- 101 238 168
- US-A- 5 931 347
- US-A1- 2015 342 411
- US-A1- 2020 290 793

## Description

### FIELD

The present disclosure relates to the field of compounding pharmaceutical compositions.

### BACKGROUND

Medical facilities, licensed pharmacists or physicians may produce individual compounded compositions by blending together various ingredients, such as one or more active pharmaceutical ingredient (API) and pharmaceutical or cosmeceutical acceptable excipients, diluent or solvents, to create a product tailored to the needs of an individual end user or patient. Such activities are commonly referred as pharmaceutical or cosmeceutical compounding. Practically speaking, in the context of pharmacy compounding, the pharmacist will typically prepare a product tailored to the needs of an individual patient based on a medical prescription.

Pharmaceutical compounding involves thorough blending of the composition ingredients, a process which often faces repeatability and quality challenges. Many mixing devices require mixing in device-specific mixing containers, which requires an additional step of decanting the compounded composition from a mixing container into a dispensing device or container. This decanting procedure increases the risk of contamination, human error, and material loss. Device-specific containers also limit the volume and/or mass of materials that can be mixed to the specifications of such containers, which is not always ideal from a practical perspective. Device-specific containers also require implementing strict cleaning/sterilization procedures to avoid cross-contamination risk when reusing the same mixing containers, which can be cumbersome and time-consuming. Alternatively, operation costs and waste are increased when containers are used and are discarded after each mixing procedure as single use mixing containers.

WO 2019/032756 A1 discloses a system and method for infusing a gas into a composition for treating skin.

### SUMMARY

This Summary is provided to introduce a selection of concepts in a simplified form that are further described below in the Detailed Description. This Summary is not intended to identify key aspects or essential aspects of the claimed subject matter.

This description relates to a container system for mixing and dispensing individual pharmaceutical or cosmeceutical compositions. The container system can be used to contain the customized ingredients requires to blend a tailored composition for a particular patient or end user. The same container in which the ingredients were mixed can then be used to store and to dispense the composition. The container system uses exchangeable lids with a universal container base that allows the ingredients to be blended together when in a mixing configuration and then dispensed in a dispensing configuration without the need to decant or transfer the mixed customized composition to a second container for use by the user. The container system can be adapted to work with a variety of mixing devices with appropriate adapters if required, and dispensing the composition from the container in dispensing configuration is easy and convenient for the end user or patient to use.

In some embodiments not part of the claims, described is a system for mixing, storage and dispensing of a cosmeceutical or pharmaceutical composition, the system comprising: i) a container comprising: a container body with a lower end and an attachment region at an upper end of the container body, and a moveable jar bottom proximate to the lower end and configured to seal an inside of the container while being moveable from a first position with respect to the container body to a second position with respect to the container body; ii) a mixing lid configured to removably attach to the attachment region; and iii) a dispensing lid configured to attach to the attachment region and thereby close the upper end, the dispensing lid comprising a dispensing pump attached to a lid body, the dispensing pump having a fluid line connecting the inside of the container at one end of the fluid line and a dispensing nozzle outside of the container at a second end of the fluid line, and wherein the dispensing pump comprises an activation button that when pressed is configured to generate a vacuum force sufficient to cause the jar bottom to move from the first position to the second position and thereby dispense a portion of a contents of the container through the dispensing nozzle.

Embodiments can include at least one of the following features: the moveable bottom can be manually moved from the first position to the second position to remove air during mixing. The moveable bottom is configured to move in response to a change of pressure within the container during dispensing. The container is airtight during dispensing. A portion of the dispensing lid is the same as a portion of the mixing lid. The mixing lid body has threads that are connectable to threads on the upper end of the container. The mixing lid body has a central nozzle configured to receive a mixing rod.

In some embodiments not part of the claims, described is a fluid dispensing container comprising: a cylindrical container body with an upper end and a lower end; a moveable jar bottom proximate to the lower end and configured to seal an inside of the container while being movable from a first position with respect to the container body to a second position with respect to the container body; a lid for closing the upper end that is detachable therefrom; and a dispensing pump attached to the lid, the dispensing pump having a fluid line connecting the inside of the container at one end and a dispensing nozzle outside of the container at a second end, wherein the dispensing pump comprises an activation button that when pressed is configured to generate a vacuum force sufficient to cause the jar bottom to move from the first position to the second position and thereby dispense a portion of a contents of the container through the dispensing nozzle.

Embodiments can include at least one of the following features: the first position is closer to the lower end and the second position is closer to the upper end. The dispensing pump comprises valves along the fluid line. The vacuum force acts to change a pressure within the container body and thereby cause the jar bottom to move. The contents of the container fill a fluid storage space between the lid and the jar bottom. A volume of the fluid storage space reduces as the contents are dispensed through the dispensing nozzle. The fluid storage space has a maximum volume of between 15 mL and 1000 mL. The portion of the contents has a volume between 0.5 mL and 2 mL. An airtight seal around a periphery of the jar bottom and an interior surface of the container body. The lid comprises threads for attaching to the container body. A removable protective cap that connects to the dispensing pump and covers the activation button. The activation button comprises a finger grip tab shaped to receive a finger.

In some embodiments not part of the claims, described is a container for storage and dispensing of a cosmeceutical or pharmaceutical composition, the container comprising: a container body with side walls that define a container interior; a jar bottom that is slidable with respect to the side walls; and a pump assembly releasably attached to an upper portion of the container body, the pump assembly in fluid communication with the container interior and having a dispensing nozzle, the pump assembly configured to change a pressure within the container interior and thereby generate a force sufficient to cause the jar bottom to slide with respect to the container body and dispense a portion of the composition through the dispensing nozzle.

Embodiments can include at least one of the following features: the container interior defines a fluid storage space. A volume of the fluid storage space reduces as the contents are dispensed through the dispensing nozzle. The fluid storage space has a maximum volume of between 15 mL and 1000 mL. The pump assembly is configured to dispense a volume between 0.5 mL and 2 mL. An airtight seal around a periphery of the jar bottom and the side walls. The container body comprises threads for attaching to a lid. The pump assembly comprises an activation button. A removable protective cap that covers the dispensing pump and covers the activation button. The activation button comprises a finger grip tab shaped to receive a finger.

In some embodiments not part of the claims, described is a container for storage and dispensing of a cosmeceutical or pharmaceutical composition, the container comprising an outer container body with side walls that define a container interior; a jar bottom configured to move from a lower position on the side walls to a raised position on the side walls; and a vacuum pump assembly releasably attached to an upper portion of the outer container body, the pump assembly in fluid communication with the container interior and having a dispensing nozzle, the pump assembly configured to generate a vacuum within the container interior and thereby generate a force sufficient to cause the jar bottom to slide from the lower position to the raised position and thereby dispense a portion of the composition through the dispensing nozzle.

Embodiments can include at least one of the following features: the container interior defines a fluid storage space. A volume of the fluid storage space reduces as a contents of the container are dispensed through the dispensing nozzle. The fluid storage space has a maximum volume of between 15 mL and 1000 mL. The vacuum pump assembly is configured to dispense a volume between 0.5 mL and 2 mL. An airtight seal around a periphery of the jar bottom and the side walls. The container body comprises threads for attaching to a lid. The vacuum pump assembly comprises an activation button. A removable protective cap that covers the activation button. The activation button comprises a finger grip tab shaped to receive a finger.

According to the invention, described is a method of mixing and storing a cosmeceutical or pharmaceutical composition, the method comprising: providing a container configured to mix ingredients that form the cosmeceutical or pharmaceutical composition contained within the container; mixing the ingredients in a mixer to form the composition; and reconfiguring the container for dispensing of the composition via a vacuum pump. Reconfiguring the container includes removing a mixing lid from a body portion of the container and attaching a dispensing lid to the body portion of the container. Mixing the ingredients can include placing the container in a program-controlled mixer.

In some embodiments not part of the claims, described is a method of mixing, storing, and dispensing a cosmeceutical or pharmaceutical composition, the method comprising placing ingredients in a container; attaching a mixing lid to the container; placing the container and attached mixing lid into a mixer for the ingredients to be mixed to form the composition; removing the container and attached mixing lid from the mixer; attaching a dispensing lid to the container; and dispensing the composition through the dispensing lid via a vacuum pump.

Advantages of the container systems described herein accrue due to their ability to hold the customized ingredients for a compounding composition as well as act as the final dispensing container from which a prepared compounded composition is to be dispensed. The advantages accrue at least in part due to the container having a moveable bottom that can be manually moved upwards (e.g., as a metering mechanism to remove air during mixing) when in a mixing configuration and can be pneumatically moved when in a dispensing configuration.

All features of exemplary embodiments which are described in this disclosure and are not mutually exclusive can be combined with one another as long as the resulting combination falls under the scope of the appended claims. Other aspects and features of the present invention will become apparent to those ordinarily skilled in the art upon review of the following description of specific embodiments in conjunction with the accompanying Figures.

### BRIEF DESCRIPTION OF THE DRAWINGS

FIG. 1 is a flow diagram of a process of preparing a personalized cosmeceutical or pharmaceutical composition in a mixing and dispensing container as described herein.
FIG. 2A shows an example system for compounding a personalized cosmeceutical or pharmaceutical composition using a blade mixer with a mixing and dispensing container as described herein.
FIG. 2B shows an example system for compounding a personalized cosmeceutical or pharmaceutical composition using superimposed revolution and rotation movements with a mixing and dispensing container as described herein.
FIG. 3A shows a side isometric view of a universal portion of a mixing and dispensing container.
FIG. 3B shows a bottom isometric view of the universal portion of the mixing and dispensing container of FIG. 3A.
FIG. 4A shows a side isometric view of a mixing and dispensing container in a mixing configuration for use with the system of FIG. 2A.
FIG. 4B shows a side isometric view of a mixing and dispensing container in a mixing configuration for use with the system of FIG. 2B.
FIG. 4C shows a side isometric view of a cap used with the mixing and dispensing container in a mixing configuration of FIG. 4A.
FIG. 5A shows a side isometric view of the mixing and dispensing container in a dispensing configuration.
FIG. 5B shows a side isometric view of the mixing and dispensing container of a second embodiment of a dispensing configuration, this second embodiment not being covered by the subject-matter of the claims.
FIG. 6 shows a side isometric view of the mixing and dispensing container in a storage configuration.

In the drawings, exemplary embodiments are illustrated by way of example. It is to be expressly understood that the description and drawings are only for the purpose of illustrating certain embodiments and are an aid for understanding. They are not intended to be a definition of the limits of the invention.

### DETAILED DESCRIPTION

This description relates to a container system for mixing and dispensing individual pharmaceutical or cosmeceutical compositions. The container system includes a container that can be used to contain the customized ingredients required to blend a tailored composition for a particular patient or end user. The same container in which the ingredients were mixed can then be used to store and to dispense the composition. The container system can use exchangeable lids with a universal container base that allows the ingredients to be blended together when in a mixing configuration and then dispensed in a dispensing configuration without the need to decant or transfer the mixed customized composition to a second container for use by the end user or patient. The container system can be adapted to work with a variety of mixing devices with appropriate adapters, if required, and dispensing the composition from the container in dispensing configuration is easy and convenient for the end user or patient to use.

### Mixing process overview

A composition of the present disclosure includes one or more ingredient that is tailored (i.e., compounded) to medical and/or cosmeceutical needs of an individual end user or patient. In some embodiments, the composition of the present disclosure can be any one of a cream, ointment, lotion, emulsion, gel, suspension, liquid solution, colloidal dispersion, or syrup. For the purpose of the present disclosure, the mixing and dispensing container acts as a metered dose device that dispenses a portion of the contained compounded composition being metered by volume. The container encloses the compounded composition, while an actuator is manually operated to cause a metering valve to allow a metered dose of the compounded composition to be dispensed at each actuation of the actuator.

In a first practical implementation, the composition of the present disclosure includes at least one active pharmaceutical ingredient (API) mixed in a pharmaceutical or cosmeceutical acceptable excipient, diluent or carrier in such a way that the composition has substantially the same API concentration throughout the volume of the composition. Such a composition can be referred to as a substantially homogeneous composition.

Substantially homogeneous compositions can be time-consuming to attain. To achieve compositions having consistent concentrations of API from one composition to another and/or consistent homogeneous API concentration within one preparation, program-controlled mixers are used. Such program-controlled mixers can have different configurations, such as a lifting blade mixer, or a planetary mixer. The mixers of different types can be used with the mixing and dispensing container systems described herein. Using the container systems, a personalized compounded pharmaceutical or cosmeceutical composition can be prepared at a medical site (e.g., a compounding pharmacy) in the same container in which the end user dispenses the composition as needed at home over time.

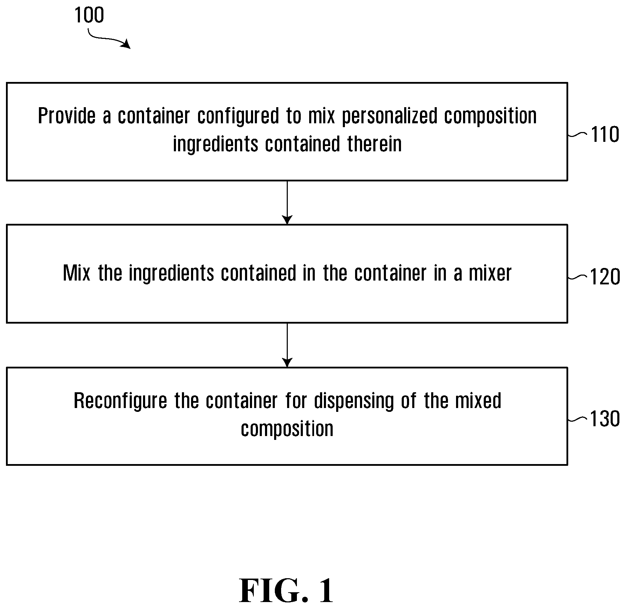

FIG. 1 is a flow chart of a general process **100** of preparing a personalized compounded pharmaceutical or cosmeceutical composition for an end user in accordance with a mixing and dispensing container of the present disclosure. In the process **100,** mixing of the ingredients that form the compounded composition are typically carried out as movements implemented in a program-controlled mixer such as a planetary mixer or vertical blade mixer.

At step **110,** the process includes providing a container (also referred to herein as a "jar") containing the composition ingredients. The container is configured for containing the composition ingredients during mixing as well as for storing and dispensing the composition once prepared. Typically, the composition ingredients include at least one API and at least one pharmaceutical or cosmeceutical acceptable excipient, diluent or carrier.

At step **120,** the process includes mixing the ingredients contained in the container in a mixer, such as a program-controlled mixer. For step **120,** the container provided in step **110** is in a mixing configuration. Mixing the ingredients using a program-controlled mixer can include obtaining pre-determined or determining operating parameters that are required to perform the machine-specific mixing movements to obtain a substantially homogeneous composition. The composition ingredients are mixed at least partly based on the parameters to produce the composition. The mixing movements disperse the API into the pharmaceutical or cosmeceutical acceptable excipient, diluent or carrier to obtain the substantially homogeneous personalized composition. Alternatively, or additionally, coarse flaky powder and fine crystalline components can be ground between friction surfaces of mixing blades and inner surfaces of the container and can thus be finely dispersed in the mixture.

At step **130,** the container is reconfigured for dispensing of the substantially homogeneous personalized composition now contained therein following the mixing step **120.** According to the invention, reconfiguring the container includes removing the container from the mixer, removing a mixing lid from the portion of the container containing the now-mixed substantially homogenous personalized composition, and attaching a dispensing lid to the container. The substantially homogeneous personalized composition contained within the mixing and dispensing container is ready to be dispensed as needed by the end user.

### Equipment for use with the mixing and dispensing containers

The mixing process carried out in step **120** may be performed in a single device such as a vertical blade mixer or a planetary mixer. Commercially available vertical blade mixers, such as the Samix^{™} mixer ES500 or U1000 from Medisca Pharmaceutique (Montreal, Canada) can be used as a mixing device. Commercially available planetary mixers, such as the MAZ^{™} mixer KK-300SS, KK-400W or KK-1000W from Medisca Pharmaceutique, can also be used as a mixing device.

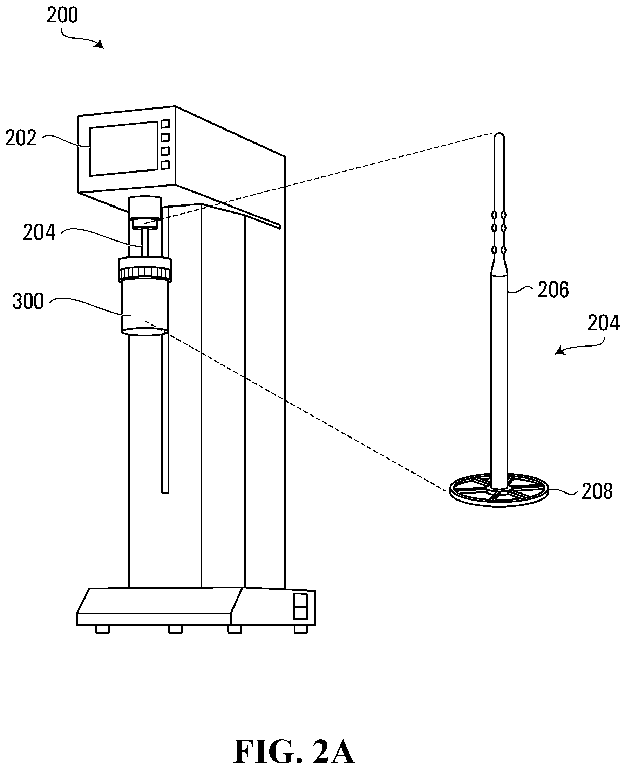

FIG. 2A shows a schematic view of an example vertical blade mixing system **200** configured to carry out the mixing movements on a mixing and dispensing container **300** held within the machine **202** to generate a substantially homogeneous personalized composition. The machine is configured so that the mixing and dispensing container **300** is held on its front side via a suitable jar holder and adaptors as needed (not shown). The mixing and dispensing container engages with a mixing unit **204** that has a mixing rod **206** affixed to a mixing blade **208.** The mixing unit **204** can be adjusted to the size of the mixing container to reach an optimum stirring result. The mixing unit **204** and machine **202** are configured such that the mixing rod **206** penetrates the mixing and dispensing container **300** via the top of the container and the mixing blade **208** is inserted into the ingredients held within the mixing and dispensing container **300.** Action of the machine **202** causes relative axial movement between the mixing blade **208** and the mixing and dispensing container **300,** that is, motion upwards and downwards. At the same time, the mixing blade **208** is rotatable and spins on the central axis of the mixing blade **208** and mixing rod **206.** The two superimposed movements, e.g., the axial and rotational movements, together cause the ingredients held within the mixing and dispensing container **300** to be mixed and/or ground.

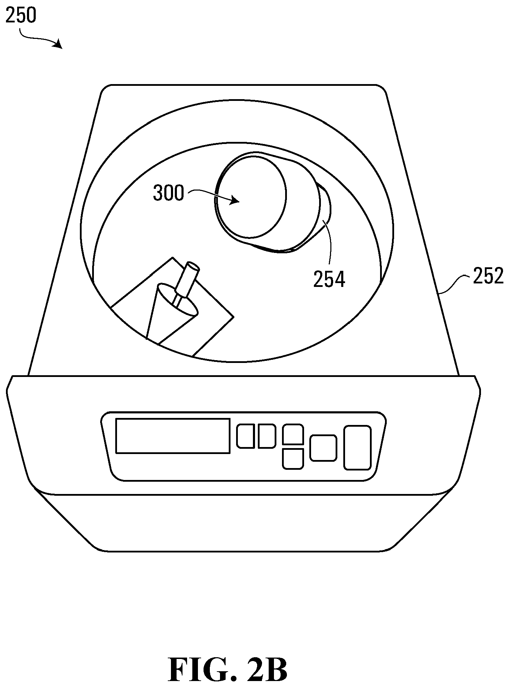

FIG. 2B shows a planetary mixing system **250** configured to carry out the mixing movements on a mixing and dispensing container **300** held in a planetary mixing machine **252** to generate a substantially homogeneous personalized composition. The planetary mixing system **250** includes the planetary mixing machine **252,** which is configured to effect superimposed revolution and rotation movements through rotation and revolution of the mixing and dispensing container **300** placed in a jar holder **254.** Generally, a planetary mixer typically includes a jar which contains the ingredients being processed arranged eccentrically on a so-called sun wheel, at a certain distance from the center of the machine. The planetary mixer is configured to impart a revolution movement to the sun wheel and a rotational movement to the jar, where the revolution movement is in an opposite direction to that of the rotation such that the ingredients contained in the container are subjected to a pattern of motion throughout space that includes superimposed revolution and rotation movements. In some instances, the mixing and dispensing container **300** can be used with various adapters that allow the mixing and dispensing container **300** to be placed directly inside the planetary mixing machine **252,** e.g., at the jar holder **254.**

### Container description

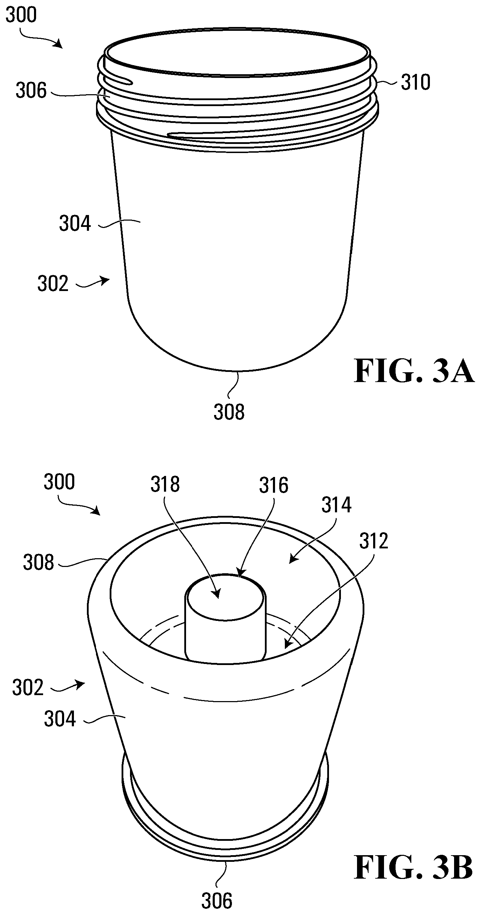

FIG. 3A shows a side isometric view of a portion of the mixing and dispensing container **300** that can be used with program-controlled mixers such as those shown in FIGS. 2A and 2B. The portion of the mixing and dispensing container **300** shown is the bottom or universal container portion **302** that is used in both the mixing configuration and the dispensing configuration.

The mixing and dispensing container **300** includes a container body **304** that has a top portion **306,** and a bottom portion **308.** The container body **304** can be generally cylindrical or may have tapered walls to form a conical-like shape, for example. The top portion **306** may have threads **310,** e.g., external threads that are sized and shaped to receive an internally threaded lid. The top portion **306** is generally open at the top such that items can be placed within the universal container portion **302** of the mixing and dispensing container **300.** That is, the ingredients that form the composition and the mixing blade **208** (from FIG. 2A) can enter the container body **304** from the open top.

FIG. 3B shows a bottom isometric view of the universal container portion **302** of the mixing and dispensing container of FIG. 3A with the bottom portion **308** in evidence. The cylindrical walls forming the container body **304** extend towards the bottom portion **308.** The bottommost surface of the bottom portion **308** has a moveable jar bottom **312.** The moveable jar bottom **312** is displaceable relative to the container body **304** such that it can slide up and down with respect to the container body **304.** For example, the moveable jar bottom **312** can move from a position nearer to the bottom portion **308** of the mixing and dispensing container **300** to a position more proximate the top portion **306,** thereby uncovering a portion of the interior wall surfaces **314** of the container body **304.** In some instances, the moveable jar bottom **312** can have a protrusion **316** extending from the central portion of the moveable jar bottom **312.** The protrusion **316** can have a divot or depression **318** sized and shaped to receive a thumb or fingertip.

In some embodiments, the mixing and dispensing container **300** may have different interior and exterior dimensions. For example, the mixing and dispensing container **300** may have an inside diameter that changes from the top of the mixing and dispensing container **300** to the bottom of the mixing and dispensing container **300,** forming a gradient. For example, the inside diameter at the top of the mixing and dispensing container **300** may be smaller than the inside diameter at the bottom of the mixing and dispensing container **300.** This gradient can be achieved in two or more sections or as a gradual taper. This gradient can, in some instances, help account for the changing volume within the mixing and dispensing container **300** as the substantially homogeneous personalized composition is dispensed therefrom.

The outside of the mixing and dispensing container **300** may be configured to be received in the particular program-controlled machine, for example, held within jar holder **254** or within an adapter within the jar holder **254.** The outside of the mixing and dispensing container **300** is sized to be held comfortably in the hand of an average adult.

The universal container portion **302** is sized and shaped to contain liquids within it. It is also configured to engage with various mixing lids including a blade mixing lid **402** or planetary mixing lid **450,** for example.

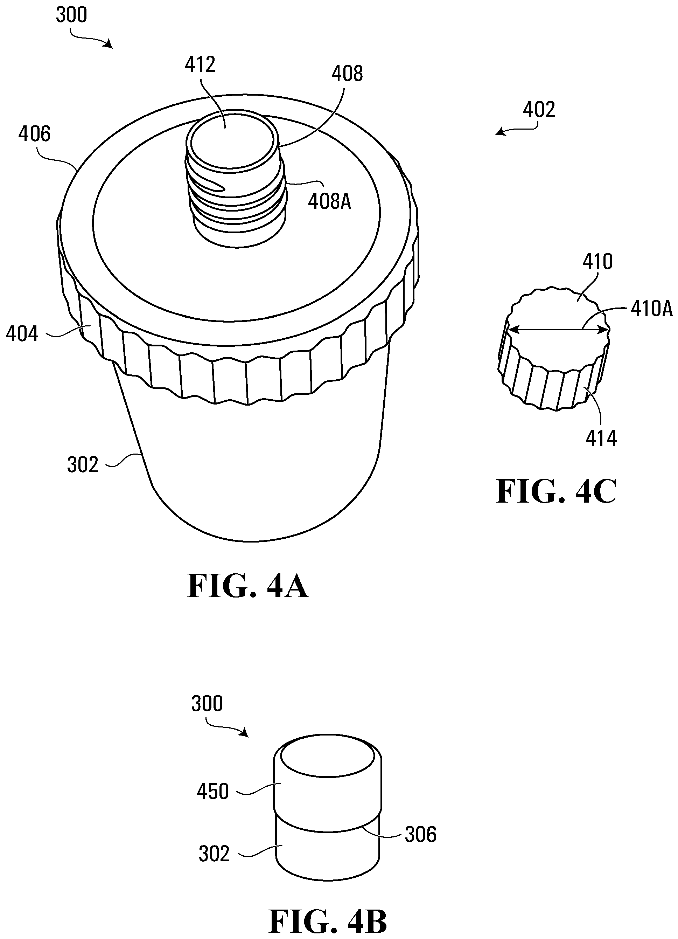

FIGS. 4A shows a side isometric view of the mixing and dispensing container **300** in a mixing configuration for use with a vertical blade mixing system **200** such as shown in FIG. 2A. In the mixing configuration shown, the universal container portion **302** of FIGS. 3A and 3B has been attached to a blade mixing lid **402.**

The blade mixing lid **402** includes a lid body **404** and a top surface **406.** The lid body **404** may have internal threads (not shown). The threads on the lid body **404** are complimentarily threads to the threads **310** of the universal container portion **302,** allowing the blade mixing lid **402** to be screwed and unscrewed therefrom.

The top surface **406** of the blade mixing lid **402** includes a nozzle **408.** The nozzle **408** has an aperture **412** that allows fluid (e.g., air or compounded composition) to escape the interior of the mixing and dispensing container **300.** The nozzle **408** may include an external thread **408A** to receive an optional internally threaded cap **410.** The cap **410** is shown in FIG. 4C. The cap **410** has an external diameter denoted **410A.** The cap **410** can also have gripping features **414** that make it easier for a user to grasp and remove the cap **410** from the rest of the blade mixing lid **402.**

To mix a composition using the vertical blade mixing system **200** of FIG. 2A, the cap **410** is removed from the nozzle **408** and the blade mixing lid **402** removed from the universal container portion **302.** The mixing rod **206** (shown in FIG. 2A) is threaded onto the blade mixing lid **402** by inserting it through the nozzle **408** via the aperture **412** from underneath the top surface **406.** With the mixing rod **206** threaded through the nozzle **408** the mixing blade **208** extends downward (away from the top surface **406**) and the end of the mixing rod **206** opposite to the mixing blade **208** is connected to a motor of the mixing unit **204.**

Meanwhile, the universal container portion **302** is filled with the composition ingredients to be mixed. With the mixing blade **208** threaded through the blade mixing lid **402** the blade mixing lid **402** is secured onto the universal container portion **302,** e.g., threads on the lid body 404 are screwed onto the threads **310** of the universal container portion **302.** The superimposed axial and rotational relative movements of the vertical blade mixing system **200** then mix the ingredients within the universal container portion **302** into the substantially homogeneous personalized composition. The nozzle **408** thus acts as a guide and support for the mixing rod **206** and can also mount the mixing and dispensing container **300** on the front side of the vertical blade mixing system **200** via e.g., a lifting arm of the vertical blade mixing system **200.**

Once the superimposed axial and rotational movements of the mixing unit **204** are complete, the mixing rod **206** can be removed in one of two ways. In the first, the user unscrews the blade mixing lid **402** from the universal container portion **302** and removes the mixing rod **206** from the nozzle **408.** This procedure is necessary for non-disposable mixing blades where the mixing blade **208** is permanently fixed to the mixing rod **206.** For disposable mixing blades, e.g., those that are releasably connected to the mixing rod **206,** it is possible to detach the mixing blade **208** from the mixing rod **206** while the mixing blade **208** is still within the universal container portion **302.** For example, the user can rotate the mixing rod **206** in the direction opposite to the one in which the mixing rod **206** normally turns to unlock the mixing rod **206** from the mixing blade **208,** which releases the mixing blade **208** and allows the mixing rod **206** to be pulled out of the nozzle **408.** In this fashion, the blade remains in the universal container portion **302** and the mixing rod **206** is removed without the need to remove the blade mixing lid **402** from the universal container portion **302.**

FIG. 4B shows a side isometric view of a mixing and dispensing container **300** in a mixing configuration for use with the planetary mixing system **250** of FIG. 2B. In this mixing configuration, the planetary mixing lid **450** can be a plain cover that that attaches to the top portion **306** of the universal container portion **302.** The planetary mixing lid **450** may be a screw-on lid, having internal threads (not shown) that are complimentarily threads to the threads **310** of the universal container portion **302,** allowing the planetary mixing lid **450** to be screwed onto and unscrewed therefrom. In some embodiments, the planetary mixing lid **450** may be a snap on lid, for example. The mixing and dispensing container **300** with the planetary mixing lid **450** attached is configured to fit within the jar holder **254** (of FIG. 2B) or within an adapter within the jar holder **254** within the planetary mixing machine **252.**

The planetary mixing lid **450** is separated from the universal container portion **302** and ingredients to be mixed placed with the universal container portion 302. The planetary mixing lid **450** is attached to the universal container portion (e.g., by screwing together or snapping together) and the filled container **300** placed within the jar holder **254** of the planetary mixing machine **252.** The ingredients contained within are subjected to superimposed revolution and rotation movements through rotation and revolution of the mixing and dispensing container **300** until a substantially homogenous composition is produced.

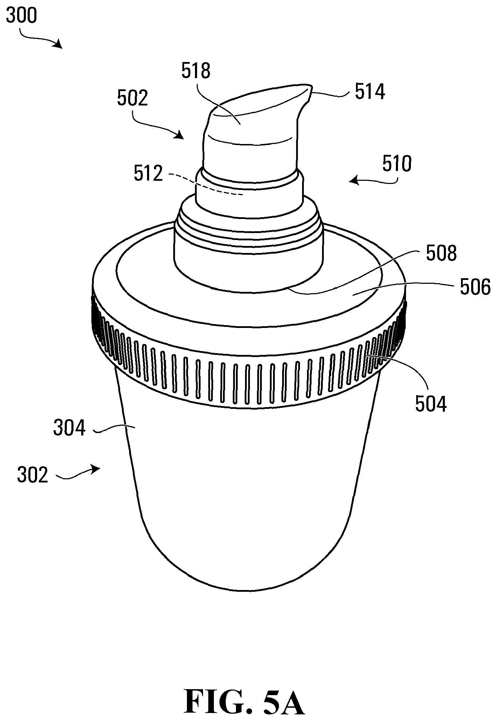

FIG. 5A shows a side isometric view of the mixing and dispensing container **300** in a dispensing configuration. In the dispensing configuration shown, the universal container portion **302** of FIGS. 3A and 3B has been attached to a dispensing lid **502.**

The dispensing lid **502** includes a lid body **504** and a top surface **506.** The lid body **504** may have internal threads (not shown). The internal threads on the lid body **504** are complimentarily to the threads **310** of the universal container portion **302,** allowing the dispensing lid **502** to be screwed and unscrewed therefrom. The top surface **506** can have a central aperture **508** that connects to a dispensing pump **510.** When the dispensing lid **502** is connected to the universal container portion **302,** the material in the interior of the mixing and dispensing container **300** (e.g., the substantially homogeneous personalized composition) can travel through the central aperture **508** into the dispensing pump **510** and out to the environment.

The dispensing pump **510** is attached to the top surface **506** of the dispensing lid **502.** In some embodiments, the dispensing pump **510** is a vacuum pump with a fluid line **512** that connects the inside of the mixing and dispensing container **300** at one end and a dispensing nozzle **514** outside of the container at the other end.

The dispensing pump **510** has an activation button **518** that when pressed generates a vacuum force sufficient to cause the moveable jar bottom **312** to move from a lower position (e.g., closer to the container's bottom portion **308**) to a more raised position (further from the container's bottom portion **308** and closer to the container's top portion **306**) and thereby dispense a portion of a contents of the container through the dispensing nozzle **514.** The dispensing pump **510** has suitable valves along the fluid line **512** as is known in the art, so that when the activation button **518** is pressed (e.g., via a finger grip tab shaped to receive a fingertip) a metered volume of the composition exits the interior of the container and is dispensed. The interior of the mixing and dispensing container **300** remains airtight during dispensing.

The change in pressure within the interior of the mixing and dispensing container **300** caused by pressing the activation button is sufficient to cause the moveable jar bottom **312** (shown in FIG. 3B) to move, e.g., to rise. This pressure change initiated by the activation button **518** is sufficient to overcome the forces keeping the moveable jar bottom **312** in place. The most important of these resistance forces keeping the moveable jar bottom in place is the frictional force between the edges of the moveable jar bottom **312** and the interior wall surfaces **314** of the container (shown in FIG. 3B). The pressure change caused by the dispensing pump **510** generates an upwards force sufficient to overcome the frictional force, while at the same time the seal between the edges of the moveable jar bottom **312** and the interior wall surfaces **314** of the container is airtight. Various features can be employed to keep the frictional forces low. For example, the moveable jar bottom **312** and/or the interior wall surfaces **314** can be made of a low-friction material or have a low-friction coating on the surfaces where the two components touch when assembled. The contact area between the edges of the moveable jar bottom **312** and the interior wall surfaces **314** of the container can be made as small as possible. A lubricant can be applied the moveable jar bottom **312** and/or the interior wall surfaces **314.**

Once mixing in a mixing configuration is completed, the dispensing lid **502** can be attached. The user can then push up on the moveable jar bottom **312** via the protrusion **316** to remove any headspace, e.g., any air remaining at the top of the mixing and dispensing container **300** following the mixing process. As the compounded contents are pushed upwards via the moveable jar bottom **312,** they reach the lid (e.g., the bottom surface of the lid opposite the top surface 506). The dispensing pump **510** can be installed and primed. Only a few pumping cycles should suffice for the dispensing pump **510** to start dispensing the composition from within the mixing and dispensing container 300.

In the dispensing configuration, the volume of the mixing and dispensing container **300** is minimized so that the contents within the container (e.g., the substantially homogeneous personalized composition) substantially completely fills the interior of a fluid storage space between the dispensing lid **502** and the moveable jar bottom **312.** The volume of that fluid storage space gradually reduces as the contents are dispensed through the dispensing nozzle. For example, the fluid storage space can have a maximum volume of between about 15 mL and about 1000 mL. That maximum volume reduces as the contents of the container are dispensed, e.g., in doses or increments between about 0.5 mL and about 2 mL.

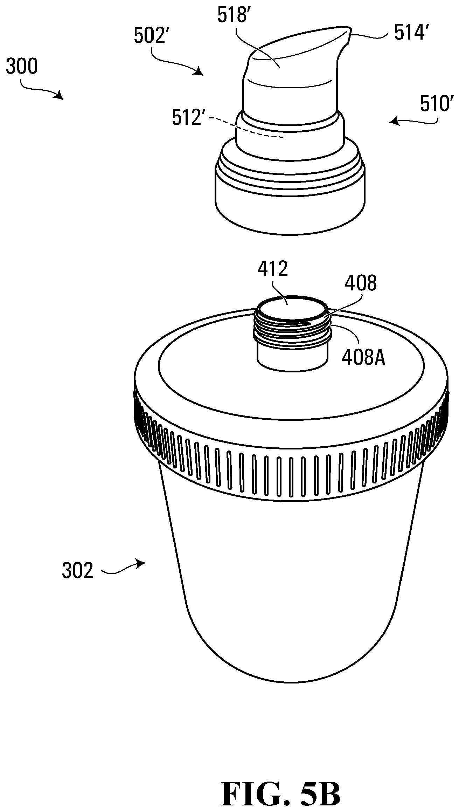

FIG. 5B shows an embodiment not part of the claims, of a dispensing lid **502'** that can place the mixing and dispensing container **300** in a dispensing configuration with fewer parts needed in the dispensing lid **502'** than those shown in FIG. 5A. The dispensing lid **502'** includes only a dispensing pump **510'** (e.g., a vacuum pump) with a fluid line **512'** that connects the inside of the mixing and dispensing container **300** at one end and a dispensing nozzle **514'** outside of the container at the other end. The dispensing pump **510'** has internal threads (not shown) that are complimentarily threads to the external thread **408A** of the nozzle 408 of the blade mixing lid **402.** The dispensing lid **502'** does not include the lid body **504** and top surface **506** as shown in FIG. 5A; to place the mixing and dispensing container **300** into a dispensing configuration the universal container portion **302** and the blade mixing lid **402** are not detached and a separate dispensing lid **502** screwed onto the universal container portion **302.** Instead, the dispensing pump **510'** is threaded onto the nozzle **408** of the blade mixing lid **402** so as to be airtight. When the dispensing pump **510'** is connected to the universal container portion **302** via the nozzle **408,** the material in the interior of the mixing and dispensing container **300** (e.g., the substantially homogeneous personalized composition) can travel via the aperture **412** through the dispensing pump **510'** and out to the environment at the dispensing nozzle **514'.**

In other embodiments, the dispensing pump **510** is permanently mounted to the dispensing lid **502** and the mixing and dispensing container **300.** That is, the mixing and dispensing container **300** is mounted to the vertical blade mixing system **200** so that during the mixing process, the dispensing pump **510** faces down. After the mixing operation, the user simply places the moveable jar bottom **312** within the universal container portion **302.** For such a configuration, suitable adapters would allow mounting the mixing and dispensing container **300** in the upside-down orientation to the vertical blade mixing system **200,** with an aperture to accommodate the mixing rod. For example, the threads **310** shown in FIG. 3A could be placed at the bottom of the container rather than the top, or in addition to at the top.

The various parts of the mixing and dispensing container **300** for the mixing configuration and dispensing configuration, being the universal container portion **302,** the blade mixing lid **402** and/or the planetary mixing lid **450,** and the dispensing lid **502** may be made of high-density polyethylene (HDPE) or polypropylene, for example.

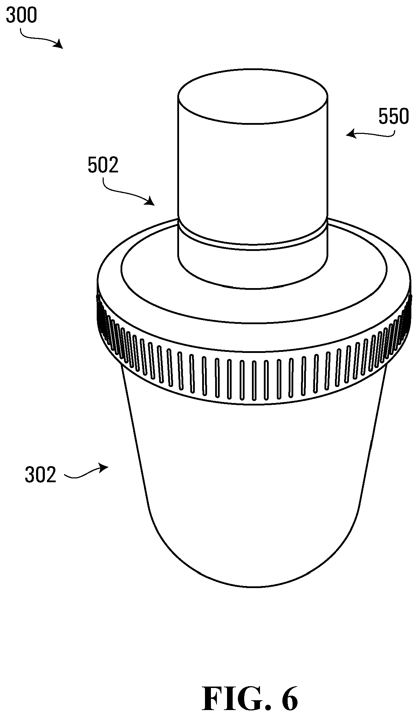

Referring to FIG. 6, the mixing and dispensing container **300** can have a storage configuration for storing the substantially homogeneous personalized composition when the composition is not being dispensed. A removable protective cap **550** can snap around the dispensing pump **510** or **510'** and prevent accidental depression of the activation button **518.**

The moveable jar bottom **312** of the mixing and dispensing container **300** can move up and down with respect to the rest of container as the system switches between modes where a composition is being, stored, and dispensed. Mechanical stops such as raised ridges or projections can prevent the moveable jar bottom **312** from moving past a final position towards the bottom portion **308** and/or the top portion **306.** In some instances, the moveable jar bottom **312** can be manually moved up and down. For example, in mixing mode with a vertical blade mixing system **200** (as shown in FIG. 4A), the moveable jar bottom **312** can be moved downwards by the action of the mixing rod **206** and the mixing blade **208.** In another example, the moveable jar bottom **312** can be manually moved upwards by pressing on the protrusion **316** on the moveable jar bottom **312.** The moveable jar bottom **312** can also be manually moved as a metering mechanism to remove the air during mixing, as air can leave the interior of the mixing and dispensing container **300** via the aperture 412 of the nozzle **408** of the blade mixing lid **402.** During any of these axial motions, the edges of the moveable jar bottom **312** maintain an airtight seal with the inside of the container while being moved between positions. The airtight seal can be maintained with a gasket, O-ring, or other type of seal that surrounds the edges of the moveable jar bottom **312.** In configurations where the inside diameter changes from the top of the mixing and dispensing container **300** to the bottom of the mixing and dispensing container **300,** the seal or gasket can be configured to expand or shrink to account for the change in the area due to the gradient. For example, the seal or gasket can be made of a compressible rubber material that fits between the moveable jar bottom **312** and/or the interior wall surfaces **314.** As the moveable jar bottom 312 slides with respect to the interior wall surfaces **314** the rubber material can expand to maintain contact with the interior wall surfaces **314.**

### Definitions

Compounding activities, in the context of the present specification, applies to combining, mixing or altering ingredients for a cosmetical or pharmaceutical composition which may include active over the counter (OTC) ingredients or prescription pharmaceutical ingredients. Within the context of the present specification, OTC and prescription ingredients are encompassed by the expression "active pharmaceuticals ingredients" (i.e., "API").

Examples of active pharmaceuticals ingredients (APIs) include, but are not limited to, antibiotics, analgesics, vaccines, anticonvulsants; antidiabetic agents, antifungal agents, antineoplastic agents, antiparkinsonian agents, anti-rheumatic agents, appetite suppressants, biological response modifiers, cardiovascular agents, central nervous system stimulants, contraceptive agents, dietary-supplements, vitamins, minerals, lipids, saccharides, metals, amino acids (and precursors), nucleic acids and precursors, contrast agents, diagnostic agents, dopamine receptor agonists, erectile dysfunction agents, fertility agents, gastrointestinal agents, hormones, immunomodulators, antihypercalcemia agents, mast cell stabilizers, muscle relaxants, nutritional agents, ophthalmic agents, osteoporosis agents, psychotherapeutic agents, parasympathomimetic agents, parasympatholytic agents, respiratory agents, sedative hypnotic agents, skin and mucous membrane agents, smoking cessation agents, steroids, sympatholytic agents, urinary tract agents, uterine relaxants, vaginal agents, vasodilator, anti-hypertensive, hyperthyroid, anti-hyperthyroid, anti-asthmatics and vertigo agents. For example, a T3/T4 medication.

In certain embodiments, the API is an extract or portion of a cannabis plant, such as a cannabinoid, a terpene, and the like. The two cannabinoids usually produced in greatest abundance are cannabidiol (CBD) and tetrahydrocannabinol (THC), although there are several dozen different cannabinoids that have been isolated from cannabis plants.

For the purpose of the present disclosure, the pharmaceutical or cosmeceutical acceptable excipient, diluent or carrier may be a semi-solid (more or less viscous fluid) or fluid (for example a cream or an emulsion). The person of skill will appreciate that pharmaceutical or cosmeceutical acceptable excipients, diluents or carriers are known in the art and may include, but without being limited thereto, anti-adherents such as magnesium stearate; binders, such as saccharides and their derivatives (sucrose, lactose, starches, cellulose or modified cellulose, sugar alcohols such as xylitol, sorbitol or maltitol), proteins such as gelatins, synthetic polymers such as polyvinylpyrrolidone (PVP) or polyethylene glycol (PEG); coloring dyes or fragrance; glidants such as fumed silica, talc, and magnesium carbonate; hydrophilic or hydrophobic lubricants such as talc or silica, and fats, e.g. vegetable stearin, magnesium stearate or stearic acid; preservatives such as antioxidant vitamins or synthetic preservatives like parabens; sorbents or other desiccant; vehicles that serve as a medium for conveying the active ingredient such as petrolatum, gum base gelatin, dimethyl sulfoxide and mineral oil or commercial products such as VersaPro^{™} Gel, HRT^{™} Cream, OleaBase^{™} Plasticized, PLO Gel Mediflo^{™}, Oral Mix^{™} or VersaPro^{™} cream, all from Medisca Pharmaceutique (Canada).

For the purpose of the present disclosure, the compounding compositions of the present description may be adapted for oral, rectal, vaginal, topical, urethral, ocular, or transdermal administration.

Those skilled in the art will appreciate that further modifications can be made without departing from the scope of the invention, which is defined by the claims appended hereto.

## Claims

1. A method of mixing and storing a cosmeceutical or pharmaceutical composition, the method comprising:
• providing a container (300) configured to mix ingredients that form the cosmeceutical or pharmaceutical composition contained within the container (300);
• mixing the ingredients in a mixer to form the composition;
• and reconfiguring the container (300) for dispensing of the composition via a vacuum pump,
**characterized in that**
reconfiguring the container (300) comprises removing a mixing lid (402; 450) from a body portion of the container (300) and attaching a dispensing lid (502) to the body portion (304) of the container (300).

2. The method of claim 1, wherein mixing the ingredients comprises placing the container (300) in a program-controlled mixer.

3. The method of claim 1 or 2, the container (300) comprising after reconfiguring:
• as body portion a cylindrical container body (304) with an upper end and a lower end;
• a moveable jar bottom (312) proximate to the lower end and configured to seal an inside of the container while being movable from a first position with respect to the container body (304) to a second position with respect to the container body (304);
• the dispensing lid (502) for closing the upper end that is detachable therefrom; and
• a dispensing pump (510) attached to the dispensing lid (502), the dispensing pump (510) having a fluid line connecting the inside of the container body (304) at one end and a dispensing nozzle (514) outside of the container body (304) at a second end,
wherein the dispensing pump (510) comprises an activation button (518) that when pressed is configured to generate a vacuum force sufficient to cause the jar bottom (312) to move from the first position to the second position and thereby dispense a portion of a contents of the container through the dispensing nozzle (514).

4. The method of claim 3, wherein the first position is closer to the lower end and the second position is closer to the upper end.

5. The method of claim 3 or 4, wherein the dispensing pump (510) comprises valves along the fluid line.

6. The method of any one of claims 3 to 5, wherein the vacuum force acts to change a pressure within the container body (304) and thereby causes the jar bottom (312) to move.

7. The method of any one of claims 3 to 6, wherein the contents of the container fill a fluid storage space between the dispensing lid (502) and the jar bottom (312).

8. The method of claim 7, wherein a volume of the fluid storage space reduces as the contents are dispensed through the dispensing nozzle (514).

9. The method of claim 7 or 8, wherein the fluid storage space has a maximum volume of between 15 mL and 1000 mL.

10. The method of any one of claims 3 to 9, wherein the portion of the contents has a volume between 0.5 mL and 2 mL.

11. The method of any one of claims 3 to 10, comprising an airtight seal around a periphery of the jar bottom (312) and an interior surface of the container body (304).

12. The method of any one of claims 3 to 11, wherein the dispensing lid (502) comprises threads for attaching to the container body.

13. The method of any one of claims 3 to 12, comprising a removable protective cap (550) that connects to the dispensing pump (510) and covers the activation button (518).

14. The method of any one of claims 3 to 13, wherein the activation button (518) comprises a finger grip tab shaped to receive a finger.

## Patentansprüche

1. Verfahren zum Mischen und Aufbewahren einer kosmezeutischen oder pharmazeutischen Zusammensetzung, wobei das Verfahren umfasst:
• Bereitstellen eines Behälters (300), der zum Mischen von Inhaltsstoffen konfiguriert ist, die die im Behälter (300) enthaltene kosmezeutische oder pharmazeutische Zusammensetzung bilden;
• Mischen der Inhaltsstoffe in einem Mixer, um die Zusammensetzung zu bilden;
• und Umkonfigurieren des Behälters (300) zur Abgabe der Zusammensetzung über eine Vakuumpumpe,
**dadurch gekennzeichnet, dass**
das Umkonfigurieren des Behälters (300) das Entfernen eines Mischdeckels (402; 450) von einem Körperabschnitt des Behälters (300) und das Anbringen eines Abgabedeckels (502) an dem Körperabschnitt (304) des Behälters (300) umfasst.

2. Verfahren nach Anspruch 1, wobei das Mischen der Inhaltsstoffe das Platzieren des Behälters (300) in einem programmgesteuerten Mixer umfasst.

3. Verfahren nach Anspruch 1 oder 2, wobei der Behälter (300) nach der Umkonfiguration Folgendes umfasst:
• als Körperabschnitt einen zylindrischen Behälterkörper (304) mit einem oberen Ende und einem unteren Ende;
• einen beweglichen Gefäßboden (312) in der Nähe des unteren Endes, der so konfiguriert ist, dass er das Innere des Behälters abdichtet, während er aus einer ersten Position in Bezug auf den Behälterkörper (304) in eine zweite Position in Bezug auf den Behälterkörper (304) bewegbar ist;
• den Abgabedeckel (502) zum Verschließen des oberen Endes, der davon abnehmbar ist; und
• eine an dem Abgabedeckel (502) angebrachte Abgabepumpe (510), wobei die Abgabepumpe (510) eine Flüssigkeitsleitung aufweist, die an einem Ende das Innere des Behälterkörpers (304) mit einer Abgabedüse (514) außerhalb des Behälterkörpers (304) an einem zweiten Ende verbindet,
wobei die Abgabepumpe (510) einen Aktivierungsknopf (518) umfasst, der beim Drücken so konfiguriert ist, dass er eine Vakuumkraft erzeugt, die ausreicht, um den Gefäßboden (312) aus der ersten Position in die zweite Position zu bewegen und dadurch einen Teil eines Inhalts des Behälters durch die Abgabedüse (514) abzugeben.

4. Verfahren nach Anspruch 3, wobei die erste Position näher am unteren Ende und die zweite Position näher am oberen Ende liegt.

5. Verfahren nach Anspruch 3 oder 4, wobei die Abgabepumpe (510) Ventile entlang der Flüssigkeitsleitung umfasst.

6. Verfahren nach einem der Ansprüche 3 bis 5, wobei die Vakuumkraft dazu dient, einen Druck innerhalb des Behälterkörpers (304) zu ändern und dadurch eine Bewegung des Gefäßbodens (312) zu bewirken.

7. Verfahren nach einem der Ansprüche 3 bis 6, wobei der Inhalt des Behälters einen Flüssigkeitsspeicherraum zwischen dem Abgabedeckel (502) und dem Gefäßboden (312) füllt.

8. Verfahren nach Anspruch 7, wobei sich ein Volumen des Flüssigkeitsspeicherraums verringert, wenn der Inhalt durch die Abgabedüse (514) abgegeben wird.

9. Verfahren nach Anspruch 7 oder 8, wobei der Flüssigkeitsspeicherraum ein maximales Volumen zwischen 15 ml und 1000 ml aufweist.

10. Verfahren nach einem der Ansprüche 3 bis 9, wobei der Teil des Inhalts ein Volumen zwischen 0,5 ml und 2 ml aufweist.

11. Verfahren nach einem der Ansprüche 3 bis 10, das eine luftdichte Abdichtung um einen Umfang des Gefäßbodens (312) und eine Innenfläche des Behälterkörpers (304) umfasst.

12. Verfahren nach einem der Ansprüche 3 bis 11, wobei der Abgabedeckel (502) Gewinde zum Anbringen an den Behälterkörper umfasst.

13. Verfahren nach einem der Ansprüche 3 bis 12, das eine abnehmbare Schutzkappe (550) umfasst, die mit der Abgabepumpe (510) verbunden ist und den Aktivierungsknopf (518) abdeckt.

14. Verfahren nach einem der Ansprüche 3 bis 13, wobei der Aktivierungsknopf (518) eine Fingergrifflasche umfasst, die so geformt ist, dass sie einen Finger aufnehmen kann.

## Revendications

1. Procédé de mélange et de stockage d'une composition cosméceutique ou pharmaceutique, le procédé comprenant :
• la fourniture d'un récipient (300) configuré pour mélanger des ingrédients qui forment la composition cosméceutique ou pharmaceutique contenue au sein du récipient (300) ;
• le mélange des ingrédients dans un mélangeur pour former la composition ;
• et la reconfiguration du récipient (300) pour la distribution de la composition par l'intermédiaire d'une pompe à vide,
**caractérisé en ce que**
la reconfiguration du récipient (300) comprend le retrait d'un couvercle de mélange (402 ; 450) d'une partie corps du récipient (300) et la fixation d'un couvercle de distribution (502) à la partie corps (304) du récipient (300).

2. Procédé selon la revendication 1, dans lequel le mélange des ingrédients comprend le placement du récipient (300) dans un mélangeur commandé par programme.

3. Procédé selon la revendication 1 ou 2, le récipient (300) comprenant après la reconfiguration :
• en tant que partie corps, un corps de récipient cylindrique (304) avec une extrémité supérieure et une extrémité inférieure ;
• un fond de pot mobile (312) à proximité de l'extrémité inférieure et configuré pour sceller un intérieur du récipient tout en étant mobile d'une première position par rapport au corps de récipient (304) à une seconde position par rapport au corps de récipient (304) ;
• le couvercle de distribution (502) pour fermer l'extrémité supérieure qui est détachable de celui-ci ; et
• une pompe de distribution (510) fixée au couvercle de distribution (502), la pompe de distribution (510) ayant une conduite de fluide reliant l'intérieur du corps de récipient (304) au niveau d'une extrémité et une buse de distribution (514) à l'extérieur du corps de récipient (304) au niveau d'une seconde extrémité,
dans lequel la pompe de distribution (510) comprend un bouton d'activation (518) qui, lorsqu'il est enfoncé, est configuré pour générer une force de vide suffisante pour amener le fond de pot (312) à entrer en mouvement de la première position à la seconde position et ainsi distribuer une partie d'un contenu du récipient à travers la buse de distribution (514).

4. Procédé selon la revendication 3, dans lequel la première position est plus proche de l'extrémité inférieure et la seconde position est plus proche de l'extrémité supérieure.

5. Procédé selon la revendication 3 ou 4, dans lequel la pompe de distribution (510) comprend des soupapes le long de la conduite de fluide.

6. Procédé selon l'une quelconque des revendications 3 à 5, dans lequel la force de vide agit pour modifier une pression au sein du corps de récipient (304) et provoque ainsi le mouvement du fond de pot (312).

7. Procédé selon l'une quelconque des revendications 3 à 6, dans lequel le contenu du récipient remplit un espace de stockage de fluide entre le couvercle de distribution (502) et le fond de pot (312).

8. Procédé selon la revendication 7, dans lequel un volume de l'espace de stockage de fluide diminue à mesure que le contenu est distribué à travers la buse de distribution (514).

9. Procédé selon la revendication 7 ou 8, dans lequel l'espace de stockage de fluide a un volume maximal compris entre 15 mL et 1000 mL.

10. Procédé selon l'une quelconque des revendications 3 à 9, dans lequel la partie du contenu a un volume compris entre 0,5 mL et 2 mL.

11. Procédé selon l'une quelconque des revendications 3 à 10, comprenant un joint étanche à l'air autour d'une périphérie du fond de pot (312) et d'une surface intérieure du corps de récipient (304).

12. Procédé selon l'une quelconque des revendications 3 à 11, dans lequel le couvercle de distribution (502) comprend des filetages pour se fixer au corps de récipient.

13. Procédé selon l'une quelconque des revendications 3 à 12, comprenant un capuchon de protection amovible (550) qui se relie à la pompe de distribution (510) et recouvre le bouton d'activation (518).

14. Procédé selon l'une quelconque des revendications 3 à 13, dans lequel le bouton d'activation (518) comprend une languette de préhension pour doigt façonnée pour recevoir un doigt.
